(19) Europäisches Patentamt
European Patent Office
Office européen des brevets

(11) Publication number: **0 054 756**
**B1**

(12) # EUROPEAN PATENT SPECIFICATION

(45) Date of publication of patent specification: **23.07.86**

(21) Application number: **81109889.6**

(22) Date of filing: **25.11.81**

(51) Int. Cl.[4]: **C 07 D 239/49,**
A 61 K 31/505,
C 07 D 239/30,
C 07 D 239/42,
C 07 D 295/14,
C 07 C 43/205, C 07 C 121/70,
C 07 C 121/75, C 07 C 121/76,
C 07 C 149/32

(54) **Antibacterial benzylpyrimidines.**

(30) Priority: **27.11.80 GB 8038146**

(43) Date of publication of application:
**30.06.82 Bulletin 82/26**

(45) Publication of the grant of the patent:
**23.07.86 Bulletin 86/30**

(84) Designated Contracting States:
**BE CH DE FR GB IT LI NL SE**

(58) References cited:
**EP-A-0 006 987**
**GB-A-1 223 881**
**GB-A-1 413 454**
**US-A-4 258 045**

(73) Proprietor: **THE WELLCOME FOUNDATION LIMITED**
**183-193 Euston Road**
**London NW1 2BP (GB)**

(72) Inventor: **Roth, Barbara**
**7 Lone Pine Road**
**Chapel Hill, North Carolina 27514 (US)**
Inventor: **Rauckman, Barbara Seavey**
**2024 Sohi Drive**
**Durham, North Carolina 27703 (US)**

(74) Representative: **Sandmair, Kurt, Dr. et al**
**Patentanwälte Schwabe, Sandmair, Marx**
**Stuntzstrasse 16**
**D-8000 München 80 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 054 756 B1

Courier Press, Leamington Spa, England.

**Description**

The present invention relates to 2,4-diamino-5-benzyl-pyrimidines, to pharmaceutical compositions containing them, to processes for preparing them and their compositions, to intermediates useful in the preparation of the compounds of the present invention and to the compounds use in the treatment of bacterial infections.

Certain 2,4-diamino-5-benzylpyrimidines have been demonstrated to exhibit useful pharmaceutical properties and to be sulphonamide potentiators. Thus, U.K. Patent Specification No. 875,562 discloses *inter alia* 2,4-diamino-5-benzylpyrimidines wherein the phenyl ring is substituted by three $C_{1-4}$alkoxy groups. Trimethoprim, 2,4-diamino-5-(3,4,5-trimethoxybenzyl)pyrimidine, is specifically disclosed in U.K. Patent No. 875,562 and is the most active general antibacterial agent amongst the 2,4-diamino-5-benzylpyrimidines known to date. Trimethoprim has been used extensively over the last decade in human therapy in combination with various sulphonamides, and in particular with sulphamethoxazole, for the treatment of *Neisserie gonorrhea* (Rodin and Seth, *Brit. J. Vener. Dis.* 1972, *48,* 517). Trimethoprim has also been extensively used in veterinary medicine for the treatment of bacterial infections. One particularly noticeable exception has been in the treatment of mastitis, where the activity of trimethoprim against the main causative organism, *Straphylococcus aureus,* is in sufficient to provide a complete inhibition of the infection.

2,4-Diamino-5-benzylpyrimidines having substituted alkoxy groups in the 4-position of the phenyl ring and possessing antibacterial activity are disclosed in Belgian Patent No. 812,375 and have the formula (I):

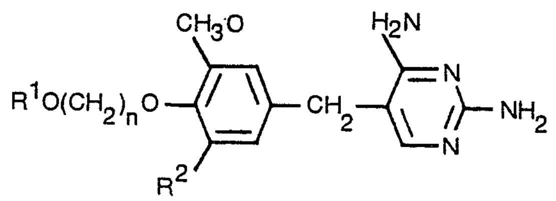

(I)

wherein n is 1, 2 or 3, $R^1$ is a $C_{1-3}$alkyl group and $R^2$ is a hydrogen or halogen atom or a $C_{1-3}$alkyl or a $C_{1-3}$alkoxy group.

U.K. Patent No. 1,223,881 discloses a group of 2,4-diamino-5-benzylpyrimidines having an aralkoxy group in the 4-position of the phenyl ring which possess antibacterial activity. Thus, U.K. Patent No. 1,223,881 discloses *inter alia* the compounds of the formula (II):

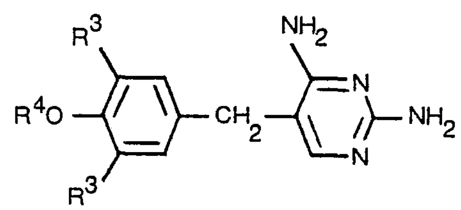

(II)

wherein the groups $R^3$ are hydrogen or halogen atoms or $C_{1-4}$alkyl or alkoxy groups and $R^4$ is an aralkyl group optionally substituted by one or more $C_{1-4}$alkyl or alkoxy groups. However, only 2,4-diamino-5-(3,5-dimethoxy-4-benzyloxybenzyl)pyrimidine is specifically exemplified out of this class of compounds.

Belgian Patent No. 751,790 discloses further structurally related compounds having antibacterial activity which include *inter alia* compounds of the formula (III):

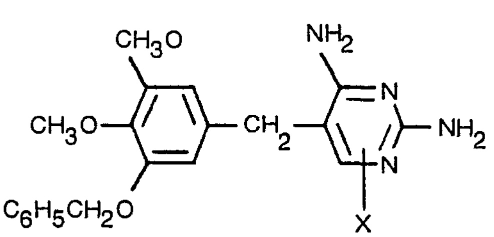

(III)

wherein X is an oxygen atom attached to one of the ring nitrogen atoms.

It has now been found that a hitherto undisclosed group of 2,4-diamino-5-benzylpyrimidines has a very high level of activity against the bacteria *Neisseria gonorrhea* and *Neisseria meningitidis.* Some of the compounds of this group are many times more active *in vitro* against *Neisseria gonorrhea* than trimethoprim. This discovery has extremely important implications in the field of human therapy in view of the recent discovery (New Scientist, 28th April 1977) of penicillin resistant strains of *Neisseria*; penicillin being widely used in the treatment of *Neisseria gonorrhea.* Many of these compounds also have a high

level of activity against the gram positive bacterial organisms *Staphylococcus aureus* and *Streptococcus agalactiae*, and *ubevis* that in many cases considerably exceeds the activity of trimethoprim against these organisms.

Accordingly, the present invention provides a compound of the formula (IV):

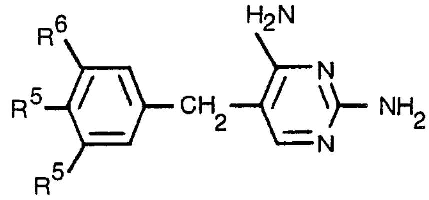

(IV)

or an acid addition salt thereof, wherein one of the groups $R^5$ is a group $O(CH_2)_nYR^7$ wherein Y is an oxygen or sulphur atom or a methylene, $OCH_2$ or $SCH_2$ group, n is an integer from 1 to 5 and $R^7$ is a phenyl group optionally substituted by one or more substituents chosen from $C_{1-4}$alkoxy, $C_{1-4}$alkyl, trifluoromethyl, nitro, amino, methylamino, dimethylamino or acetamido groups; the other group $R^5$ and $R^6$ being the same or different, each being hydrogen, halogen, $C_{2-4}$alkenyl, $C_{2-4}$alkenyloxy, nitro, cyano, hydroxy, benzyloxy, $C_{1-4}$alkyl or $C_{1-4}$alkoxy, the alkyl or alkoxy group being optionally substituted by halogen, hydroxy or $C_{1-2}$alkoxy, amino optionally substituted by one or two $C_{1-4}$alkyl or $C_{1-4}$acyl groups or the nitrogen atom forming part of a five or six membered heterocyclic ring, a group $—OSO_2R^8$ or $S(O)rR^8$ wherein $R^8$ is $C_{1-3}$alkyl and r is 0, 1, 2, a group $—COR^9$ wherein $R^9$ is methyl, ethyl, methoxy, ethoxy, amino, methylamino, ethylamino, dimethylamino or diethylamino.

A preferred group of compounds of the formula (IV) is that wherein one of the groups $R^5$ is a group $—O(CH_2)_nYR^7$ wherein Y is an oxygen or sulphur atom or a methylene, $OCH_2$ or $SCH_2$ group n is an integer from 1 to 5 and $R'$ is a phenyl group optionally substituted by one or more substituents chosen from $C_{1-4}$alkoxy, $C_{1-4}$alkyl, trifluoromethyl, nitro, amino, methylamino, dimethylamino or acetamido groups. The substituents will normally be at the meta or para positions of the phenyl ring.

The other group $R^5$ is a hydrogen or a halogen atom or a hydroxy, benzyloxy, $C_{1-4}$alkoxy, $C_{1-4}$alkyl or $C_{2-4}$alkenyl group, and $R^6$ is a hydrogen or halogen atom or a hydroxy, $C_{1-4}$alkoxy, $C_{1-4}$alkyl or $C_{3-4}$alkenyl group.

Preferably n is an integer from 2 to 4. $R^6$ is suitably an iodine atom or methoxy, ethoxy, ethyl, propyl, propenyl or allyl group and is preferably a methoxy group. The group $R^5$ which is not the group $O(CH_2)_nYR^7$ is suitably an iodine atom or a methoxy, ethoxy, ethyl, propyl, propenyl or allyl group and is preferably a methoxy group.

In one preferred embodiment of the present invention Y is a methylene, $OCH_2$ or $SCH_2$ group.

One preferred group of compounds of the present invention is that of formula (V):

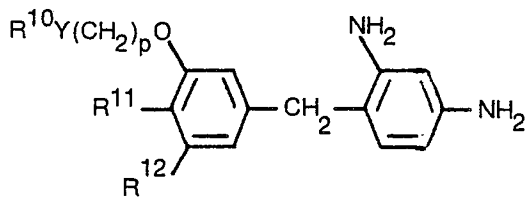

(V)

or acid addition salts thereof, wherein p is 2 or 3, Y is an oxygen or sulphur atom or a methylene group, $R^{10}$ is a benzyl group or a phenyl group optionally substituted by a $C_{1-4}$alkoxy group, $R^{11}$ is an iodine atom or a methoxy, ethoxy, or ethyl group and $R^{12}$ is an iodine atom or a methoxy, ethoxy, ethyl, propyl, propenyl or allyl group.

A further preferred group of compounds of the present invention is that of the formula (VI):

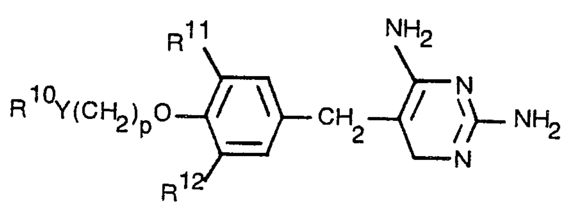

(VI)

or acid addition salts thereof, wherein p, Y, $R^{10}$, $R^{11}$ and $R^{12}$ are as defined in relation to formula (V).

A further preferred group of compounds of the present invention is that of the formula (VII):

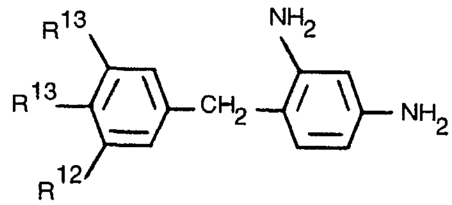

(VII)

or an acid addition salt thereof wherein one of the groups $R^{13}$ is a group $O(CH_2)_{p-1}YCH_2R^{10}$ and the other group $R^{13}$ is a group $R^{11}$; $R^{10}$, $R^{11}$, $R^{12}$, p and Y being as defined in relation to formula (V). Preferably the group $O(CH_2)_{p-1}YCH_2R^{10}$ is at the meta position of the phenyl ring.

The compounds of the formula (V) are preferred compounds of the present invention for treating gram positive bacterial organisms.

Preferably $R^{10}$ is a benzyl, phenyl or m-methoxy phenyl group and $R^{11}$ and $R^{12}$ are methoxy or ethyl groups and in particular methoxy groups.

Preferred compounds of the present invention include:

2,4-Diamino-5-(3,4-dimethoxy-5-(4-phenyl-n-butoxy)benzyl)pyrimidine,
2,4-Diamino-5-(3,4-dimethoxy-5-(3-(m-methoxyphenyl)-n-propoxy)benzyl)pyrimidine,
2,4-Diamino-5-(3,4-dimethoxy-5-(3-phenyl-n-propoxy)benzyl)pyrimidine,
2,4-Diamino-5-(3,4-dimethoxy-5-(4-(p-aminophenyl)-n-butoxy)benzyl)pyrimidine,
2,4-Diamino-5-(3,4-dimethoxy-5-(4-(p-nitrophenyl)-n-butoxy)benzyl)pyrimidine,
2,4-Diamino-5-(3,4-dimethoxy-5-(2-benzyloxyethoxy)benzyl)pyrimidine,
2,4-Diamino-5-(3,4-dimethoxy-5-(4-phenoxy-n-butoxy)benzyl)pyrimidine,
2,4-Diamino-5-(3,4-dimethoxy-5-(2-phenylthio)ethoxy)benzyl)pyrimidine,
2,4-Diamino-5-(3,4-dimethoxy-5-(2-(benzylthio)ethoxy)benzyl)pyrimidine.

Suitable acid addition salts of the compounds of the formula (IV) include those formed with both organic and inorganic acids. Such acid addition salts will normally be pharmaceutically acceptable. Thus, preferred salts include those formed from hydrochloric, sulphuric, citric, tartaric, phosphoric, lactic, benzoic, glutamic, pyruvic, acetic, succinic, fumaric, maleic, oxaloacetic, isethionic, stearic, methanesulphonic, toluene.p.sulphonic, lactobionic and glucuronic acids.

In a further aspect, the present invention provides a pharmaceutical composition comprising a compound of the formula (IV) in combination with a pharmaceutically acceptable carrier. By the terms, "pharmaceutical composition" and "pharmaceutically acceptable carrier" is meant those compositions and carriers suitable for use in human and/or veterinary medicine.

The compounds of the formula (IV) can conveniently be presented in the compositions of the present invention in an effective unit dosage form, that is to say in an amount sufficient to be effective against the bacterial organism *in vivo*.

The pharmaceutically acceptable carriers present in the compositions of the present invention are materials recommended for the purpose of administering the medicament. These may be liquid, or solid or gaseous materials, which are otherwise inert or medically acceptable and are compatible with the active ingredients.

These pharmaceutical compositions may be given parenterally, orally, used as a suppository, applied as an ophthalmic solution, or applied topically as an ointment, cream or powder. However, oral and parenteral administration of the compositions is preferred for human use. For veterinary use, intramammary as well as oral and parenteral administration is preferred.

For oral administration, fine powders or granules will contain diluting, dispensing and/or surface agents, and may be presented in a draught, in water or in a syrup, in capsules or cachets in the dry state or in a non-aqueous suspension wherein suspending agents may be included, or in a suspension in water or syrup. Where desirable or necessary, flavouring, preserving, suspending, thickening or emulsifying agents can be included. Tablets are a particularly suitable form for oral administration and will contain conventional excipients such as granulating, disintegrating and lubricating agents.

For parenteral administration, the compounds may be present in sterile aqueous injection solutions which may contain antioxidants or buffers.

As stated above, free base or a salt thereof may be administered in its pure form unassociated with other additives, in which case, a capsule or cachet is the preferred carrier.

Other compounds which may be included are, for example, medically inert ingredients, e.g. solid and liquid diluents, such as lactose, glucose, starch or calcium phosphate for tablets or capsules; olive oil or ethyl oleate for soft capsules; and water or vegetable oil for suspensions or emulsions; lubricating agents such as talc or magnesium stearate; gelling agents, such as colloidal clays; thickening agents such as gum tragacanth or sodium alginate; and other therapeutically acceptable accessory ingredients such as humectants, preservatives, buffers, and antioxidants which are useful as carriers in such formulations.

Alternatively the active compound may be presented in a pure form as an effective unit dosage, for instance, compressed as a tablet or the like.

For veterinary use, different intramammary formulations will normally be prepared for use in dry cows

and for use in milking cows. Thus, formulations for dry cow use will normally in in an oil, such as peanut oil, gelled with a gelling base such as aluminium monostearate. Formulations for milking cow use will usually include an emulsifying agent (for example Tween® 20 or a polysorbate) and a milk miscible carrier such as peanut oil or a mineral oil.

It may be advantageous to include the compounds of formula (IV) in a pharmaceutical composition which includes other active ingredients for example p-aminobenzoic acid competitors such as sulphonamides.

Of known p-aminobenzoic acid competitors, the following sulphonamide compounds (or pharmaceutically acceptable salts thereof) are particularly useful:

Sulfanilamide, Sulfadiazine, Sulfamethisazole, Sulfapyridine, Sulfathiazole, Sulfamerazine, Sulfamethazine, Sulformethoxine, 2-(p-Aminobenzene)sulfonamido-3-methoxypyrazine (Kelfizina), p,p'-Diamino-diphenylsulfone, Amino-*p*-toluenesulfonamide, 5-Sulfanilamido-2,4-dimethyl pyrimidine, 4-(N'-Acetylsulfanilamido)-5,6-dimethoxy pyrimidine, 3-Sulfanilamido-4,5-dimethyl isoazole, 4-Sulfanilamido-5-methoxy-6-decyloxy pyrimidine sulfamonomethoxine, 4-*p*-(8-hydroxy-quinilinyl-4-azo)-phenyl sulfanilamido-5,6-dimethoxy pyrimidine, Sulfadimethoxine, Sulfadimidine, Sulfamoxole, Sulfadoxine, Sulfaguanidine, Sulfathiodimethoxine, Sulfamethoxazole, Sulfaquinoxaline and *p*-(2-Methyl-8-hydroxy-quinolinyl-5-azo)-phenyl sulfanilamido-5,6-dimethoxy pyrimidine.

However, the most preferred combinations include those containing Sulfadiazine, Sulfamethoxazole, Sulfadoxine, Sulfamoxole or Sulfadimidine. The ratio of the compound of the formula (IV) to sulphonamide will normally be from 3:1 to 1:10, for example 1:1 to 1:5. A particularly preferred composition of the present invention comprises a compound of formula (IV) and sulfadiazine in a ratio of 1:1 to 1:4, preferably 1:2, together with a pharmaceutically acceptable carrier. A further preferred composition comprises a compound of the formula (IV) and sulfamethoxazole in a ratio of 1:2 to 1:6.

Tablets or other forms of presentation provided in discrete units may conveniently contain an amount of compound of the formula (IV) which is effective at a dosage or as a multiple of the same, for instance, for human use, units containing 2.5 to 200 mg usually around 30 to 100 mg; for veterinary use units containing 30 to 500 mg. Intramammary presentations will normally contain 50 to 500 mg, preferably 100 to 200 mg, of the compound of the formula (IV) for use in the milking cow and 100 to 1000 mg, preferably 200 to 600 mg, for use in the dry cow.

The pharmaceutical compositions of the present invention can be prepared by the admixture of a compound of the formula (IV) with a pharmaceutically acceptable carrier. Other active ingredients, such as a sulfonamide, or conventional pharmaceutical excipients may be admixed as required.

Still another aspect of the present invention provides a method for the treatment or prophylaxis of bacterial infections in mammals by the administration of an effective non-toxic antibacterial amount of a compound of the formula (IV) or a pharmaceutically acceptable salt thereof, or a composition as hereinbefore described.

As indicated above, the compounds of the formula (IV) are generally useful in treating such infections by topical application or by oral administration or injection. The compounds of the formula (IV) are normally administered at a dose from 0.1 mg/kg to 30 mg/kg per day. The dose range for adult humans is generally from 25 to 300 mg/day and preferably 50 to 100 mg/day. The dose range for intramammary administration of the compounds of the formula (IV) is generally from 100 to 500 mg, preferably 200 to 400 mg, per quarter of the udder per day to milking cows and from 100 to 1000 mg, preferably 200 to 500 mg, per quarter of the udder to dry cows. Milking cows will normally receive four to six medications of a composition of the present invention, a dose being conveniently administered at milking time (i.e. twice daily) to each of the desired quarters of the udder. Dry cows will normally receive only one medication of a composition of the present invention, one dose being provided to each of the four quarters of the udder.

The compounds of the formula (IV) and their pharmaceutically acceptable salts may be prepared by several routes.

Thus, the present invention provides a process for the preparation of compounds of formula (IV) as defined hereinbefore which process comprises (a) (i) the reaction of a compound of formula (VIII):

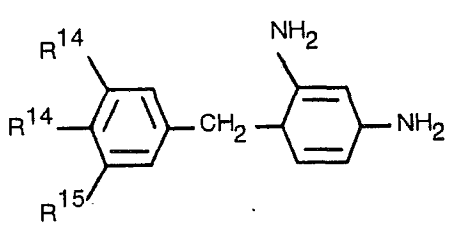

(VIII)

wherein one group $R^{14}$ is a hydroxy group whilst the other group $R^{14}$ and $R^{15}$ are the same or different and each is a group $R^6$ as hereinbefore defined other than hydroxy with a compound of the formula $R^7Y(CH_2)_nU$ wherein n, Y and $R^7$ are as hereinbefore defined and U is a leaving group, such as a halogen atom or the anionic residue of a carboxylic or sulphonic acid, for example methane sulphonic acid or toluene-p-sulphonic acid;

(ii) the reaction of guanidine as a guanidine salt with a compound of the formula (IX) or (X):

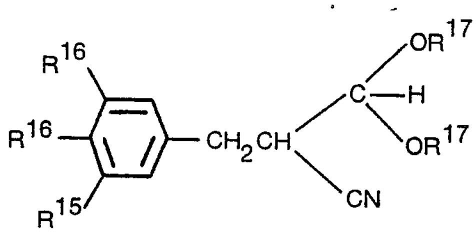

(IX)

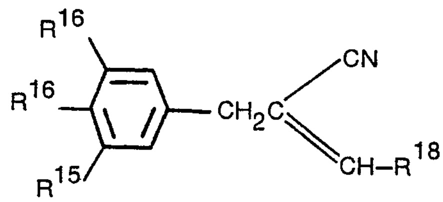

(X)

wherein $R^{15}$ is as hereinbefore defined, one group $R^{16}$ is a group $O(CH_2)_nYR^7$ as hereinbefore defined and the other group $R^{16}$ is a group $R^6$ as hereinbefore defined other than hydroxy, $R^{17}$ is a $C_{1-4}$alkyl group and $R^{18}$ is a $C_{1-4}$alkoxy group or an amino, $C_{1-4}$alkylamino, benzylamino, di-$(C_{1-4})$alkylamino, naphthylamino, optionally substituted anilino, piperidino or N-methyl piperazino group and most preferably $R^{18}$ is an anilino group;

(iii) the reaction of a compound of the formula (XI):

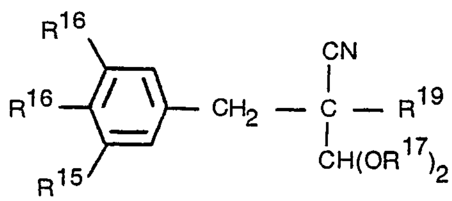

(XI)

wherein $R^{15}$, $R^{16}$, and $R^{17}$ are as hereinbefore defined and $R^{19}$ is an alkoxycarbonyl or aldehyde group, with potassium or sodium hydroxide in a $C_{1-4}$alkanol followed by addition of guanidine; and thereafter in (i), (ii) and (iii) when one or both $R^{15}$ and $R^{16}$ is benzyloxy optionally converting this to hydroxy by conventional methods known in the art, for example by catalytic hydrogenation;

b. when $R^6$ and one of $R^5$ are not halogen atoms, the reaction of a compound of formula (XII):

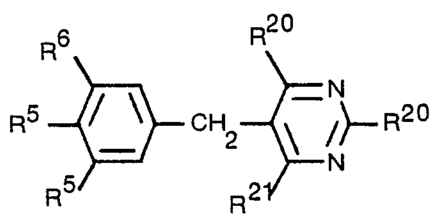

(XII)

wherein $R^{20}$ is an amino group or a leaving group, such as $C_{1-4}$alkylthio group or a halogen atom, $R^{21}$ is a hydrogen or halogen atom, except that both groups $R^{20}$ cannot be amino groups and $R^5$ and $R^6$ are as hereinbefore defined other than halogen with an aminating agent such as ammonia and thereafter when $R^{21}$ is a halogen atom removing this by hydrogenolysis;

c. when the group $R^5$ at the *p*-position of the phenyl ring is a hydroxy group; the reaction of a compound of the formula (XIII):

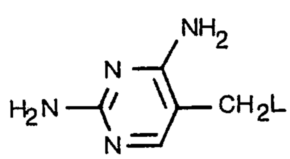

(XIII)

wherein L is a leaving group, with a compound of the formula (XIV):

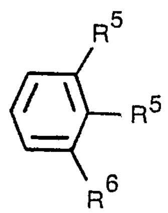

(XIV)

wherein the $R^5$ group adjacent to $R^6$ is a hydroxy group and the other groups are as defined in relation to formula (IV):

d. the reaction of a compound of the formula (XV):

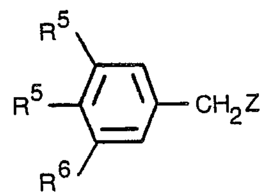

(XV)

wherein Z is a hydroxy or di-substituted amino group or a halogen atom and $R^5$ and $R^6$ are as hereinbefore defined, with a compound of the formula (XVI):

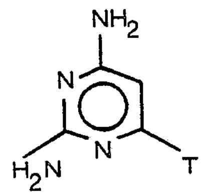

(XVI)

wherein T is a hydrogen or halogen atom or a hydroxy, $C_{1-4}$alkylthio, $C_7$—$C_{11}$-aralkylthio or mercapto group provided that if the group $R^5$ at the *p*-position of the phenyl ring is not a hydroxy group, T is a hydroxy group; in the case where T is a halogen atom or an alkylthio or aralkylthio or mercapto group, this is then removed by hydrogenolysis and in the case where T is a hydroxy group, this is then converted to halogen and removed by hydrogenolysis;

e. when Y is not an alkylene group, the reaction of a compound of the formula (XVII):

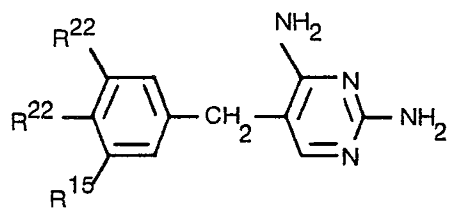

(XVII)

wherein one group $R^{22}$ is a group $—O(CH_2)_nQ$ whilst the other group $R^{22}$ is a group $R^6$ as hereinbefore defined other than hydroxy; $R^{15}$ is as hereinbefore defined and Q is a halogen atom for example a chlorine atom with a compound of the formula $R^7PH$ wherein $R^7$ is as hereinbefore defined and P is an oxygen or sulphur atom or an $CH_2O$ or $CH_2S$ group, and thereafter when $R^{15}$ is a benzyloxy group optionally converting this to a hydroxy group by conventional methods known in the art, for example by catalytic hydrogenation;

f. the conversion of one group $R^5$, $R^6$, to another $R^5$, $R^6$ by methods known *per se*, for example the alkylation or benzylation of a compound of the formula (IV) wherein $R^5$ and/or $R^6$ are hydroxy groups to give the corresponding alkoxy, benzyloxy compound under conventional conditions or the hydrolysis of a compound of the formula (IV) wherein a group $R^5$ and/or $R^6$ is a benzyloxy group to give the corresponding compound of the formula (IV) wherein $R^5$ and/or $R^6$ is a hydroxy group under conventional conditions.

The reaction of the compound of the formula (VIII) with the compound of the formula $R^7Y(CH_2)_nU$ can conveniently be carried out in an aprotic solvent, for example dimethyl formamide, diethylsulphoxide or hexamethylphosphoramide (HMPA) at a non-extreme temperature, for example between $-10°C$ and $150°C$ and conveniently at room temperature, in the presence of a base, for example sodium methoxide. Alternatively, the reaction may be carried out in an alcoholic solvent, for example ethanol, at an elevated temperature, preferably at reflux, in the presence of a base, such as potassium carbonate. The

intermediates of the formula (VIII) are either known compounds or can be prepared by methods known in the art for preparing structurally related benzylpyrimidines having a hydroxy group in the *m*- or *p*- position of the phenyl ring.

The reaction of guanidine with a compound of the formula (IX) or (X) will take place under conditions analogous to those described in U.K. Patents Nos. 1,133,766 and 1,261,455 respectively, for the preparation of structurally related benzylpyrimidines. Again, the compounds of the formula (IX) and (X) may be prepared by methods known in the art.

The reaction of a compound of the formula (XI) with guanidine and the preparation of the compounds of the formula (XI) will be carried out by methods analogous to those described in Belgian Patent No. 855 505.

In the compounds of the formula (XII) when $R^{20}$ or $R^{21}$ are halogen atoms these are suitably chlorine or bromine atoms. The reaction may conveniently be carried out under the reaction conditions described in U.K. Patent Nos. 875,562 and 1,132,082. The reduction of $R^{21}$ when this is halogen will suitably be carried out under the conditions described in German Offenlegungsschrift 2258228. This is not a preferred method for preparing those compounds wherein $R^5$ and $R^6$ are groups that are susceptible to catalytic hydrogenolysis, for example optionally substituted benzyloxy or halogen atoms.

Suitable leaving groups L in formula (XIII) include hydroxy groups, halogen atoms such as bromine and chlorine and the anionic residues of carboxylic or sulphonic acids for example methane sulphonic acid and toluene-p-sulphonic acid. Other suitable leaving groups not exemplified in U.K. Patent No. 1,413,471 include amino groups, preferably di-$C_{1-6}$alkylamino groups. This reaction is normally carried out in the presence of a carboxylic or a strong acid, such as a sulphonhic acid.

Suitably Z is a dialkylamino or cyclic amino group containing up to 10 carbon atoms; a dimethylamino group is particularly convenient. The reaction can be carried out in the presence of base under conditions well known to those skilled in the art of Mannich reactions. It has been found that the reaction may suitably be carried out in the presence of an alkoxide ion such as methoxide ion at an elevated temperature, suitably between 100° and 200°C in a solvent having a suitably high boiling point, for example a glycol such as ethylene glycol. The dethiation is suitably carried out by hydrogenolysis in the presence of a transition metal catalyst; Raney nickel is particularly suitable for this purpose. The reaction will normally be carried out in a polar solvent, for example a $C_{1-4}$alkanol such as methanol or ethanol.

Again, this not a preferred method of preparing those compounds of the formula (V) wherein $R^5$ or $R^6$ are groups that are susceptible to a catalytic hydrogenolysis.

The reaction of a compound of the formula (XVII) with a compound $R^7PH$ will normally take place in a suitable solvent, for example a $C_{1-4}$alkanol, in the presence of a base, conveniently sodium methoxide, at a non-extreme temperature, i.e. between 0 and 170°C and conveniently between 50 and 100°C.

It will be apparent to those skilled in the art that when certain ring substituents ($R^5$ and $R^6$) are present in the final compounds of the formula (IV), certain methods of preparation will preferably not be used to make these compounds due to the possibility of the reaction conditions changing the substituent group, for example hydrogenolysis when a benzyloxy or alkenyl group is present.

The intermediates of the formula (Ix), (X), (XI), (XII), (XIV) and (XV) are novel.

In yet another aspect, the present invention provides the first pharmaceutical composition of compounds of the formula (IV) for use in human and veterinary medicine. The composition comprising the compound of the formula (IV) is preferably used in human medicine in the treatment or prophylaxis of *Neisseria* infections. The composition comprising compounds of the formula (IV) is preferably used in veterinary in the treatment or prophylaxis of mastitis, particularly in cows.

The examples give details on how particular intermediates may be prepared. Other intermediates may be prepared by analogous methods taking into account the particular functional groups involved.

The following examples illustrate the inventions:

Example 1

2,4-Diamino-5-(3,4-dimethoxy-5-(3-phenoxypropoxy)benzyl)pyrimidine

2,4-Diamino-5-(3,4-dimethoxy-5-hydroxybenzyl)pyrimidine (1.1 g, 4 mmol) was dissolved in 15 ml of dimethyl sufoxide under nitrogen. Then 2N potassium hydroxide (2 ml) was added, followed by 3-phenoxypropyl bromide (0.86 g, 4 mmol). The reaction was heated at 75°C for 1½ hour, and then the dimethyl sulfoxide was evaporated. The residue was dissolved in chloroform (150 ml) and extracted with 0.5 N sodium hydroxide. The organic solvent was removed and the product was recrystallized from absolute ethanol, giving 1.24 g (75% yield). The product was recrystallized a second time from ethyl acetate giving 0.77 g, mpt. 143—144°C.

| | | | |
|---|---|---|---|
| *Anal.* Calcd. for $C_{22}H_{26}N_4O_4$: | C, 64.37; | H, 6.38; | N, 13.65. |
| Found: | C, 64.38; | H, 6.49; | N, 13.49. |

Example 2

2,4-Diamino-5-(3,4-dimethoxy-5-(3-(m-methoxyphenyl)propoxy)benzyl)pyrimidine

This compound was made in a similar manner using 3-(m-methoxyphenyl)propyl bromide and the phenol of Example 1. The reaction gave 0.98 g, mpt. 129—131°C (ethyl acetate), 58% yield.

Example 3

2,4-Diamino-5-(3,4-dimethoxy-5-(4-phenylbutoxy)benzyl)pyrimidine

This compound was made in a similar manner using 4-phenylbutyl chloride and the phenol of Example 1. The reaction gave 1.2 g of the product, mpt. 149—150°C (ethanol), 74% yield.

*Anal.* Calcd. for $C_{22}H_{28}N_4O_3$: C, 67.63; H, 6.91; N, 13.72.
Found: C, 67.52; H, 6.98; N, 13.61.

Example 4

2,4-Diamino-5-(3,4-dimethoxy-5-(3-phenylpropoxy)benzyl)pyrimidine

This compound was made in a similar manner using 3-phenylpropyl bromide and the phenol of Example 1. The reaction gave the title compound, mpt. 151.5—152.5°C in 72% yield.

Example 5

2,4-Diamino-5-(3,5-diiodo-4-(3-phenoxypropoxy)benzyl)pyrimidine

2,4-Diamino-5-(4-hydroxybenzyl)pyrimidine (2.1 g, 9.7 mmol) was dissolved in glacial acetic acid (75 ml) and heated to 40°C. Then iodine monochloride (3.4 g, 21 mmol) in glacial acetic acid (10 ml) was added dropwise to the reaction, followed by water (70 ml). The reaction was heated at 80°C for 0.5 hour, cooled, and diluted with water (150 ml), followed by the slow addition of concentrated ammonium hydroxide (141 ml) to pH 8.5. The temperature was maintained between 20—40°. A brown precipitate separated; 3.72 g (82%). Two recrystallizations from 85% ethanol plus one equivalent of concentrated hydrochloric acid gave 2,4-diamino-5-(3,5-diiodo-4-hydroxybenzyl)pyrimidine hydrochloride (1.15 g), mp. 237—241°C.

2,4-Diamino-5-(3,5-diiodo-4-(3-phenoxypropoxy)benzyl)pyrimidine was prepared according to the procedure of Example 1, using 3-phenoxypropyl bromide (1.5 g, 7.0 mmol) and 2,4-diamino-5-(3,5-diiodo-4-hydroxybenzyl)pyrimidine (3.28 g, 7.0 mmol) in the presence of potassium tertiary butoxide (0.86 g, 7.7 mmol). The reaction gave 3.1 g (73%) of the product which was recrystallized from absolute ethanol in the presence of concentrated hydrochloric acid to give the hydrochloride mpt. 276—278°C.

*Anal.* Calcd. for $C_{20}H_{20}I_2N_4O_2.HCl$: C, 37.61; H, 3.31; N, 8.77.
Found: C, 38.27; H, 3.33; N, 8.66

Example 6

2,4-Diamino-5-(3,5-diethyl-4-(2-benzyloxyethoxy)benzyl)pyrimidine

2,4-Diamino-5-(3,5-diethyl-4-hydroxybenzyl)pyrimidine (6.8 g, 25 mmol) was dissolved in dimethyl sulfoxide (60 ml) under nitrogen, followed by sodium methylate (1.5 g, 27.8 mmol). The mixture was heated slightly until all dissolved. Then 2-benzyloxymethyl tosylate (7.65 g, 25 mmol) was added, and the reaction was heated at 100° for 1½ hour. Glacial acetic acid (0.5 ml) was added to neutralize the reaction, and the dimethyl sulfoxide was removed *in vacuo* to give a residue which was worked up as before giving 8.4 g of crude product (82.6% yield). After two recrystallizations from ethanol in the presence of an equivalent of hydrochloric acid, the product as the hydrochloride salt melted at 234—238°C.

*Anal.* Calcd. for $C_{24}H_{30}N_4O_2HCl$: C, 65.07; H, 7.05; N, 12.65; Cl, 8.00.
Found: C, 64.87; H, 7.13; N, 12.71; Cl, 7.91.

Example 7

2,4-Diamino-5-(3,4-dimethoxy-5-(3-phenylpropoxy)benzyl)pyrimidine

A solution of 2-(3,4-dimethoxy-5-(3-phenylpropoxy)benzyl)-3-morpholino acrylonitrile (143.2 g; 0.338 moles) in absolute alcohol (210 ml) was refluxed with aniline hydrochloride for ½ hour. The reaction was cooled to 25°C and guanidine hydrochloride (64.5 g, 0.675 mole) sodium methoxide (57.1 g, 1.057 mole) and absolute ethanol (190 ml) was added, then reflux continued. The product recrystallized from the cooled reaction mixture. Filtration followed by a water wash gave 2,4-diamino-5-(3,4-dimethoxy-5-(3-phenylpropoxy)benzyl)pyrimidine, (107.6 g, 81%). A sample recrystallized from ethanol then recrystallized as the acetate salt from 8% acetic acid in water, reslurried in dilute ammonium hydroxide and washed with water has mp. 154.5—156°C.

*Anal.* Calcd. for $C_{22}H_{26}N_4O_3$: C, 66.99; H, 6.64; N, 14.20.
Found: C, 66.91; H, 6.75; N, 14.28.

Example 8

2,4-Diamino-5-(3,4-dimethoxy-5-(4-(4-nitrophenyl)butoxy)benzyl)pyrimidine

2,4-Diamino-5-(5-hydroxy-3,4-dimethoxybenzyl)pyrimidine (4.14 g, 15 mmol) was dissolved in 60 ml of dimethylsulfoxide under nitrogen. Then potassium t-butoxide (1.85 g, 16.5 mmol) was added and stirred 10 minutes until precipitate formed, followed by 5.24 g (15 mmol) of 4-(4-nitrophenyl)butyl p-toluenesulfonate. The reaction was heated to 80°C for 10 minutes until the precipitate dissolved, and then stirred at room temperature for 3 hours. The dimethylfulfoxide was evaporated, and the residue was

dissolved in methylene chloride and extracted with 0.1 N sodium hydroxide. The organic solvent was removed, and the product was recrystallised from absolute ethanol, giving 5.35 g (79% yield) of title compound, mp. 157—158°C.

*Anal.* Calcd. for $C_{23}H_{27}N_5O_5$: C, 60.92; H, 6.00; N, 15.44.
Found: C, 60.89; H, 6.02; N, 15.42.

Example 9

2,4-Diamino-5-(3,4-dimethoxy-5-(2-phenoxyethoxy)benzyl)pyrimidine

This compound was made in a similar manner using 2-phenoxyethyl bromide and the phenol of Example 1 on a 5 mmol scale. The reaction needed to be heated to 100°C for 6 hour to be completed. The reaction gave 1.55 g, mp. 176—180°C (absolute ethanol), 78% yield.

*Anal.* Calcd. for $C_{21}H_{24}N_4O_4$: C, 63.62; H, 6.10; N, 14.13.
Found: C, 63.67; H, 6.11; N, 14.11.

Example 10

2,4-Diamino-5-(3,4-dimethoxy-5-(4-phenoxybutoxy)benzyl)pyrimidine hydrochloride

This compound was made in a similar manner to Example 9 using 4-phenoxybutyl bromide and the phenol of Example 1 on a 5 mmol scale. The reaction gave 1.34 g, mp. 218—220°C (absolute ethanol), 58% yield of the hydrochloride salt.

*Anal.* Calcd. for $C_{23}H_{28}N_4O_4HCl$: C, 59.93; H, 6.34; N, 12.15.
Found: C, 59.97; H, 6.34; N, 12.14.

Example 11

2,4-Diamino-5-(4-(4-aminophenyl)butoxy-4,5-dimethoxybenzyl)pyrimidine

2,4-Diamino-5-(3,4-dimethoxy-5-(4-(4-nitrophenyl)butoxybenzyl)pyrimidine (3.24 g, 7.5 mmol) was dissolved in 100 ml of absolute ethanol and reduced using 100 mg of 5% palladium on charcoal and 3.05 ml of 95% hydrazine at reflux for 3 hours. The catalyst was removed by filtration, and the solvent was evaporated to dryness. The residue was recrystallised from ethyl acetate giving 2.40 g (75% yield) of the title compound, mp. 122—123°C.

*Anal.* Calcd. for $C_{23}H_{29}N_5O_3$: C, 65.23; H, 6.90; N, 16.54.
Found: C, 54.88; H, 6.95; N, 16.43.

Example 12

2,4-Diamino-5-(3,5-dimethoxy-4-(4-(4-nitrophenyl)butoxy)benzyl)pyrimidine

This compound was made in a similar manner to Example 8 using 2,4-diamino-5-(4-hydroxy-3,5-dimethoxybenzyl)pyrimidine hydrochloride hydrate and the tosylate of Example 8 on a 10 mmol scale. The reaction needed to be heated to 100°C for 7 hour to be completed. The compound was purified on a column (silica gel, methylene chloride/methanol, 19:1) and then recrystallised from absolute ethanol giving 0.88 g (19% yield) of the title compound, mp. 178—182°C.

*Anal.* Calcd. for $C_{23}H_{27}N_5O_5$: 60.92; H, 6.00; N, 15.44.
Found: C, 60.87; H, 6.04; N, 15.42.

Example 13

2,4-Diamino-5-(4-(4-(4-aminophenyl)butoxy)-3,5-dimethoxybenzyl)pyrimidine

This compound was made in a similar manner to Example 11 using the product of Example 12. In a 1.5 mmol scale the reaction gave 0.49 g (77% yield), mp. 150—151°C (absolute ethanol).

*Anal.* Calcd. for $C_{23}H_{29}N_5O_3$: C, 65.23; H, 6.90; N, 16.54.
Found: C, 65.20; H, 6.91; N, 16.53.

Example 14

2,4-Diamino-5-(3,4-dimethoxy-5-(2-(phenylthio)ethoxy)benzyl)pyrimidine

2,4-Diamino-5-(3,4-dimethoxy-5-hydroxybenzyl)pyrimidine (5.0 g, 18.1 mmol) was dissolved in 60 ml of dimethyl sulfoxide under nitrogen. Then 2.05 g (18.3 mmol) of potassium t-butoxide was added. The mixture was stirred for ten minutes, followed by the addition of 3.5 ml (19.3 mmol) of 2-chloroethyl p-toluene sulfonate. The mixture was then stirred overnight. The dimethyl sulfoxide was removed *in vacuo* and the residue dissolved in 100 ml of methylene chloride, followed by extraction with 100 ml of 0.1 N sodium hydroxide. The organic layer was evaporated, giving 5.6 g, (91%) of crude 2,4-diamino-5-(3,4-dimethoxy-5-(2-chloroethoxy)benzyl)pyrimidine which, upon recrystallisation from ethanol, gave 2.90 g of material melting at 180—181°C.

| | | | | |
|---|---|---|---|---|
| *Anal.* Calcd. for $C_{15}H_{19}ClN_4O_3$: | C, 53.18; | H, 5.65; | N, 16.54; | Cl, 10.46. |
| Found: | C, 53.15; | H, 5.65; | N, 16.33; | Cl, 10.44. |

The above product (0.68 g, 2 mmol) was dissolved in 40 ml of ethanol, and a solution of 0.23 ml (2.2 mmol) of thiophenol and 0.13 g, (2.4 mmol) of sodium methoxide in ethanol was added under nitrogen. The reaction was refluxed for 7 hours, and the precipitate removed. The solvent was stripped off, and the residue purified on a flash column (W. Clark Still et al., J. Org. Chem. *43,* 2923 (1978)) using silica gel (0.04—0.063 mm, 230—400 mesh) and eluting with methylene chloride:methanol/15:1.

This gave 0.66 g (80.5%) of the title compound which was recrystallised from ethanol in the presence of an equivalent of hydrochloric acid to give 0.58 g, mp. 209—211°C, of 2,4-diamino-5-(3,4-dimethoxy-5-(2-phenylthioethoxy)benzyl)pyrimidine hydrochloride.

| | | | |
|---|---|---|---|
| *Anal.* Calcd. for $C_{21}H_{24}N_4O_3S.HCl$: | C, 56.18; | H, 5.61; | N, 12.48. |
| Found: | C, 56.12; | H, 5.61; | N, 12.39. |

Example 15

2,4-Diamino-5-(3,4-dimethoxy-5-(2-(benzylthio)ethoxy)benzyl)pyrimidine

This compound was made in the manner of Example 14, using benzyl mercaptan instead of thiophenol. The product was purified similarly from a flash column, which gave 0.71 g, (83%) of the title compound, which was recrystallized from ethanol, as the hydrochloride, giving 0.56 g, of material melting at 199—201°C.

| | | | |
|---|---|---|---|
| *Anal.* Calcd. for $C_{22}H_{26}N_4O_3S.HCl$: | C, 57.07; | H, 5.88; | N, 12.10. |
| Found: | C, 56.79; | H, 5.84; | N, 11.92. |

*Preparation of starting materials*

Example A

Preparation of 2,4-Diamino-5-(3,4-dimethoxy-5-hydroxybenzyl)pyrimidine

The preparation of the title compound is described in *Helvetica Chimica Acta, 53,* 945 (1970).

Example B

Preparation of 2,4-Diamino-5-(3,5-diethyl-4-hydroxybenzyl)pyrimidine

The title compound was prepared as described in Example 1 of U.K. Patent No. 1,413,471.

Example C

2-(3,4-Dimethoxy-5-(3-phenylpropoxybenzyl)-3-morpholinoacrylonitrile

3,4-Dimethoxy-5-(3-phenylpropoxy)benzaldehyde (111.3 g, 0.37 mole) in dimethyl sulfoxide (60 ml) was warmed to 65°C and added to a 65°C solution of 3-morpholino-propionitrile (60.5 g; 0.489 mole) and sodium methoxide (2.64 g, 0.049 mole) in dimethyl sulfoxide over a 5 minute interval. The reaction was warmed to 80°C and stirred for 30 minutes. After cooling to 25°C water (100 ml) was added and the mixture extracted with ether (4 × 100 ml). The combined organic layers were washed with water (3 × 100 ml), concentrated and dried by adding absolute alcohol (100 ml) and reconcentrated to give an oil, 2-(3,4-Dimethyl-5-(3-phenylpropoxy)benzyl)-3-morpholino acrylonitrile (143.2 g, 91%). NMR consistent with assigned structure as a mixture of E and Z isomers.

The $LD_{50}$s (expressed in mg/kg intraperitoneal) of two compounds of the present invention were determined in male rats. The results are as follows:

| Compound | $LD_{50}$ |
|---|---|
| 2,4-diamino-5-(3,4-dimethoxy-5-(3-phenylpropoxy)benzyl)pyrimidine | >100 |
| 2,4-diamino-5-(3,4-dimethoxy-5-(3-(m-methoxyphenyl)propoxy)benzyl)pyrimidine | >100 |

*Formulation Example*

Syringes for intramammary use in cows were prepared containing the following ingredients. Quantities given are per syringe.

| | |
|---|---|
| 2,4-diamino-5-(3,4-dimethoxy-5-(3-phenylpropoxy)benzyl)pyrimidine | 0.15 g |
| Sulfadiazine | 0.30 g |
| Glycerol monostearate | 0.38 g |
| Tween® 65 | 0.02 g |
| Arachis Oil | 3.15 g |

The glycerol monostearate, Tween 65 and Arachis Oil were mixed together and melted at 65°C. The drugs were added and the mixture homogenised using a high speed stirrer. The homogenised product was cooled to 50°C and filled into intramammary syringes under sterile conditions.

Pharmacological Activity

a. Antibacterial Activity of 2,4-diamino-5-benzyl pyrimidines of the present invention

Compound Name

In vitro Antibacterial Activity
MIC (ug/ml) compound/trimethoprim

| Compound Name | *St. pyogenes* CN 10 | *St. pyogenes* S3640 | *Strep. Faecalis* CN 478 | *Staph. aureus* CN 491 | *E. Coli* CN 314 | Act. vs TMP *N. gonorrhea* | |
|---|---|---|---|---|---|---|---|
| | | | | | | | |
| 2,4-diamino-5-(3,4-dimethoxy-5-(3-phenoxypropoxy) benzyl)pyrimidine | 0.3/0.3 | 0.3/0.3 | 0.1/0.1 | 0.1/0.3 | 1.0/0.1 | Wellcome test 4.12 | Difco test 11.28 |
| 2,4-diamino-5-(3,4-dimethoxy-5-(3-(m-methoxyphenyl) propoxy)benzyl) pyrimidine | 1.0/0.3 | 1.0/0.3 | 0.3/0.1 | 0.3/0.3 | 10/0.1 | 3.20 | 9.63 |
| 2,4-diamino-5-(3,4-dimethoxy-5-(4-phenylbutoxy)benzyl) pyrimidine | 0.1/0.3 | 0.1/0.3 | 0.1/0.1 | 0.03/0.3 | 3.0/0.1 | 1.54 | 11.35 |
| 2,4-diamino-5-(3,5-diethyl-4-(2-benzyl-oxyethoxy)benzyl pyrimidine | 3.0/0.3 | 1.0/3.0 | 0.3/0.1 | 3.0/0.3 | 10/0.1 | 0.35 | — |
| 2,4-diamino-5-(3,4-dimethoxy-5-(3-phenylpropoxy)benzyl) pyrimidine | 0.3/0.3 | 0.3/0.1 | 0.1/0.1 | 0.03/0.3 | 1.0/0.1 | 5.46 | 12.81 |
| 2,4-diamino-5-(3,4-dimethoxy-5-(2-(phenyl-thio)-ethoxy)benzyl-pyrimidine | 0.5/0.5 | 0.5/0.5 | 0.5/0.1 | 0.5/0.5 | 1.0/0.1 | — | — |
| 2,4-diamino-5-(3,4-dimethoxy-5-(2-(benzylthio)-ethoxy)-benzyl pyrimidine | 0.5/0.5 | 0.5/0.5 | 0.1/0.1 | 0.05/0.5 | 0.5/0.1 | — | — |

b. Dihydrofolate reductase Inhibitory Activity of 2,4-diamino-5-(3,5-O-substituted benzyl) pyrimidines of the present invention

| Compound Name | Dihydrofolate reductase inhibition, $I_{50} \times 10^{8}$ | | | |
|---|---|---|---|---|
| | N. gonorrhea | E. coli | Rat liver | P. berghei |
| 2,4-diamino-5-(3,4-dimethoxy-5-(3-phenoxy-propoxy)benzyl)pyrimidine | 3.2 | 3.7 | 8000 | 15 |
| 2,4-diamino-5-(3,4-dimethoxy-5-(3-(m-methoxyphenyl)propoxy)benzyl)pyrimidine | 1.8 | 5.7 | 8300 | 5.7 |
| 2-4-diamino-5-(3,4-dimethoxy-5-(4-phenyl-butoxy)benzyl)pyrimidine | 1.8 | 0.23 | 4300 | 1.1 |
| 2,4-diamino-5-(3,5-diethyl-4-(2-benzyloxy-ethoxy)benzyl)pyrimidine | 4.8 | 1.5 | 2600 | 3.4 |
| 2,4-diamino-5-(3,4-dimethoxy-5-(3-phenyl-propoxy)benzyl)pyrimidine | 1.5 | 0.48 | 9400 | 10 |
| 2,4-diamino-5-(3,4-dimethoxy-5-(benzyloxy-ethoxy)benzyl)pyrimidine | 7.8 | 0.54 | 30000 | — |
| 2,4-dimethoxy-5-(dimethoxy-5-(3-p-amino-phenylpropoxy)benzyl)pyrimidine | 2.9 | 0.72 | 8100 | — |
| 2,4-diamino-5-(3,4-dimethoxy-5-(3-p-nitro-phenylpropoxy)benzyl)pyrimidine | — | 1.7 | 2500 | — |
| 2,4-diamino-5-(3,4-dimethoxy-5-(4-phenoxy-butoxy)benzyl)pyrimidine | 1.9 | 0.4 | 7400 | — |
| 2,4-diamino-5-(3,4-dimethoxy-5-(2-phenyl-thio)ethoxy)benzyl)pyrimidine | 1.14 | 0.53 | 3700 | — |
| 2,4-diamino-5-(3,4-dimethoxy-5-(2-benzyl-thio)ethoxy)benzyl)pyrimidine | 1.8 | 0.20 | 4700 | — |
| Trimethoprim | 66. | 0.54 | 30000 | 12 |

Antibacterial activity of 2,4-diamino-5-benzylpyrimidines against bacterial strains of particular significance in bovine mastitis in-vitro activity MIC's µM/ml × $10^3$

All organisms were isolated from the bovine udder.

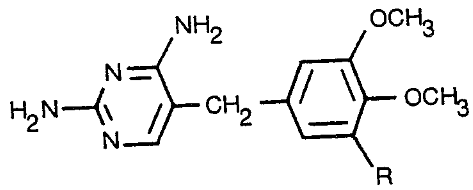

| Compound No. | R |
|---|---|
| TMP | $OCH_3$ |
| 1 | $O(CH_2)_3$ Ph |
| 2 | $O(CH_2)_3$(m-OMe) Ph |
| 3 | $O(CH_2)_4$ Ph |
| 4 | $O(CH_2)_2OCH_2$ Ph |
| 5 | $O(CH_2)_3O$ Ph |

Approximate MIC'S in µM/ml × $10^3$

| Organism | Internal Ref. No. | TMP | 1 | 2 | 3 | 4 | 5 |
|---|---|---|---|---|---|---|---|
| *Staphylococcus aureus* | 316 | 1 | 0.025 | 0.025 | 0.075 | 0.25 | 0.25 |
| " | 317 | 0.33 | 0.025 | 0.025 | 0.025 | 0.075 | 0.075 |
| " | 348 | 1 | 0.025 | 0.025 | 0.075 | 0.25 | 0.075 |
| " | 349 | 1 | 0.025 | 0.025 | 0.075 | 0.075 | 0.075 |
| " | 352 | 3.3 | 0.075 | 0.075 | 0.075 | 0.25 | 0.25 |
| " | 353 | 1 | 0.025 | 0.025 | 0.025 | 0.075 | 0.075 |
| " "OXFORD" | 6571 | 1 | 0.025 | 0.025 | 0.025 | 0.075 | — |
| *Streptococcus* | 321 | 1 | 0.075 | 0.25 | 0.25 | 0.25 | — |
| *agalactiae* | 371 | 3.3 | 0.75 | 0.75 | 0.25 | 2.5 | — |
| *Streptococcus* | 366 | 3.3 | 0.75 | 0.75 | 0.75 | 3.3 | — |
| *dysgalactiae* | 367 | 1 | 0.25 | 0.25 | 0.75 | 1 | — |
| *Streptococcus uberis* | 359 | 10 | 2.5 | 2.5 | 0.25 | 2.5 | — |

## Claims

1. A benzylpyrimidine of the general formula:

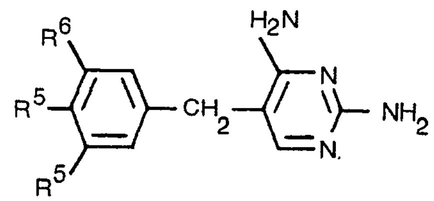

(IV)

or an acid addition salt thereof, wherein one of the groups $R^5$ is a group $O(CH_2)_nYR^7$ wherein Y is an oxygen or sulphur atom or a methylene, $OCH_2$ or $SCH_2$ group, n is an integer from 1 to 5 and $R^7$ is a phenyl group optionally substituted by one or more substituents chosen from $C_{1-4}$alkoxy, $C_{1-4}$alkyl, trifluoromethyl, nitro, amino, methylamino, dimethylamino or acetamido groups; the other group $R^5$ and $R^6$ being the same or different, each being hydrogen, halogen, $C_{2-4}$alkenyl, $C_{2-4}$alkenyloxy, nitro, cyano, hydroxy, benzyloxy, $C_{1-4}$alkyl or $C_{1-4}$alkoxy, the alkyl or alkoxy group being optionally substituted by halogen, hydroxy or $C_{1-2}$alkoxy, amino optionally substituted by one or two $C_{1-4}$alkyl or $C_{1-4}$acyl groups or the nitrogen atom forming part of a five or six membered heterocyclic ring, a group $-OSO_2R^8$ or $S(O)rR^8$ wherein $R^8$ is $C_{1-3}$alkyl and r is 0, 1, 2, a group $-COR^9$ wherein $R^9$ is methyl, ethyl, methoxy, ethoxy, amino, methylamino, ethylamino, dimethylamino or diethylamino.

2. A compound according to claim 1 wherein one of the groups $R^5$ is a group $O(CH_2)_nYR^7$ wherein Y is an oxygen or sulphur atom or a methylene, $OCH_2$ or $SCH_2$ group, n is an integer from 1 to 5 and $R^7$ is a phenyl group optionally substituted by one or more substituents chosen from $C_{1-4}$alkoxy, $C_{1-4}$alkyl, trifluoromethyl, nitro, amino, methylamino, dimethylamino or acetamido groups, the other group $R^5$ is a hydrogen or a halogen atom or a hydroxy, benzyloxy, $C_{1-4}$alkoxy, $C_{1-4}$alkyl or $C_{2-4}$alkenyl group, and $R^6$ is a hydrogen or halogen atom or a hydroxy, $C_{1-4}$alkoxy, $C_{1-4}$alkyl or $C_{2-4}$alkenyl group.

3. A benzylpyrimidine of the general formula:

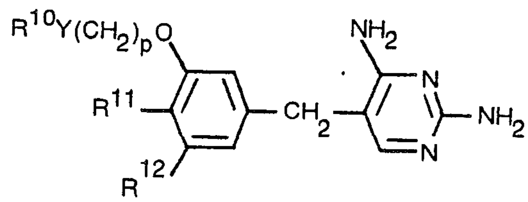

(V)

or an acid addition salt thereof, wherein
p is 2 or 3,
Y is an oxygen atom or a methylene group,
$R^{10}$ is a benzyl group or a phenyl group optionally substituted by a $C_{1-4}$alkoxy group,
$R^{11}$ is an iodine atom or a methoxy, ethoxy or ethyl group and
$R^{12}$ is an iodine atom or a methoxy, ethoxy, ethyl, propyl, propenyl or allyl group.

4. A benzylpyrimidine of the general formula:

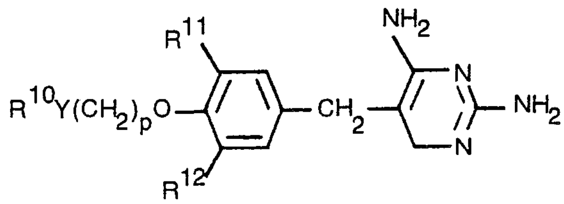

(VI)

or an acid addition salt thereof, wherein
p is 2 or 3,
Y is an oxygen atom or a methylene group,
$R^{10}$ is a benzyl group or a phenyl group optionally substituted by a $C_{1-4}$alkoxy group,
$R^{11}$ is an iodine atom or a methoxy, ethoxy or ethyl group and
$R^{12}$ is an iodine atom or a methoxy, ethoxy, ethyl, propyl, propenyl or allyl group.

5. A benzylpyrimidine of the general formula:

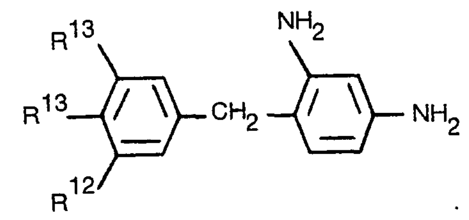

(VII)

or an acid addition salt thereof, wherein

one of the groups $R^{13}$ is a group $—O(CH_2)_{p-1}—YCH_2R^{10}$, and the other group $R^{13}$ is a group $R^{11}$, wherein $R^{10}$, $R^{11}$, $R^{12}$, p and Y are as defined in relation to formula (V) in claim 3.

6. A compound according to any one of claims 3 to 5 wherein

p is 2,

$R^{10}$ is a benzyl, phenyl or (m-methoxy)phenyl group and

$R^{11}$ and $R^{12}$ are methoxy or ethyl groups.

7. A benzylpyrimidine selected from:

2,4-Diamino-5-(3,4-dimethoxy-5-(4-phenyl-n-butoxy)benzyl)pyrimidine,
2,4-Diamino-5-(3,4-dimethoxy-5-(3-(m-methoxyphenyl)-n-propoxy)benzyl)pyrimidine,
2,4-Diamino-5-(3,4-dimethoxy-5-(3-phenyl-n-propoxy)benzyl)pyrimidine,
2,4-Diamino-5-(3,4-dimethoxy-5-(4-(p-aminophenyl)-n-butoxy)benzyl)pyrimidine,
2,4-Diamino-5-(3,4-dimethoxy-5-(4-(p-nitrophenyl-n-butoxy)benzyl)pyrimidine,
2,4-Diamino-5-(3,4-dimethoxy-5-(2-benzyloxyethoxy)benzyl)pyrimidine,
2,4-Diamino-5-(3,4-dimethoxy-5-(4-phenoxy-n-butoxy)benzyl)pyrimidine,
2,4-Diamino-5-(3,4-dimethoxy-5-(2-phenylthio)ethoxy)benzyl)pyrimidine,
2,4-Diamino-5-(3,4-dimethoxy-5-(2-(benzylthio)ethoxy)benzyl)pyrimidine,

or an acid addition salt thereof.

8. A pharmaceutical composition comprising a benzylpyrimidine of the formula (IV), as defined in claim 1 herein, in combination with a pharmaceutically acceptable carrier.

9. A pharmaceutical composition according to claim 8 wherein the composition additionally comprises a sulphonamide which is a p-aminobenzoic acid competitor.

10. A pharmaceutical composition according to claim 8 or claim 9 which comprises a benzylpyrimidine of the formula (IV), as defined in claim 1 herein, and sulfadiazine in a 1:2 ratio.

11. A pharmaceutical composition according to claim 8 or claim 9 which comprises a benzylpyrimidine of the formula (IV), as defined in claim 1 herein, and sulfamethoxazole in a ratio of 1:2 to 1:6.

12. A benzylpyrimidine of the formula (IV), as defined in claim 1 herein, for use in human and veterinary medicine.

13. A process for the preparation of a benzylpyrimidine of the formula (IV) as defined in claim 1 herein, which process comprises

(a) (i) the reaction of a compound of the formula (VIII):

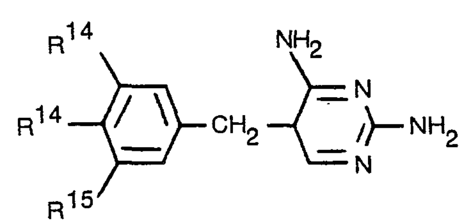

(VIII)

wherein one group $R^{14}$ is a hydroxy group whilst the other group $R^{14}$ and $R^{15}$ are the same or different, and each is a group $R^6$ as hereinbefore defined in claim 1 other than hydroxy, with a compound of the formula $R^7Y(CH_2)_nU$ wherein n, Y and $R^7$ are as hereinbefore defined in claim 1 and U is a leaving group;

(ii) the reaction of guanidine as a guanidine salt with a compound of the formula (IX) or (X):

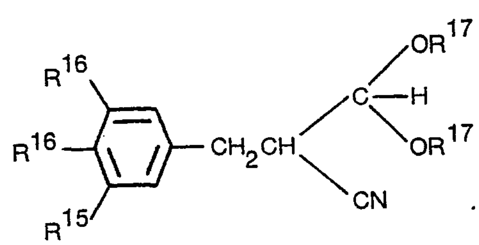

(IX)

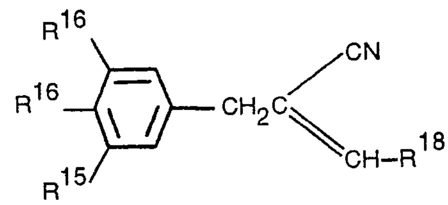

(X)

wherein $R^{15}$ is as hereinbefore defined, one group $R^{16}$ is a group $—O(CH_2)_nYR^7$ as hereinbefore defined in claim 1 and the other group $R^{16}$ is a group $R^6$ as hereinbefore defined in claim 1, $R^{17}$ is a $C_{1-4}$alkyl group and $R^{18}$ is a $C_{1-4}$alkoxy group or an amino, $C_{1-4}$alkylamino, benzylamino, di-$(C_{1-4})$alkylamino, naphthylamino, optionally substituted anilino, piperidino or N-methyl piperazino group;
optionally followed by the conversion of one group $R^5$, $R^6$ to another group $R^5$, $R^6$ by methods known per se.

14. A process for the preparation of a benzylpyrimidine of the formula (IV) as defined in claim 1 herein, but wherein Y is not an alkylene group, which process comprises the reaction of a compound of the formula

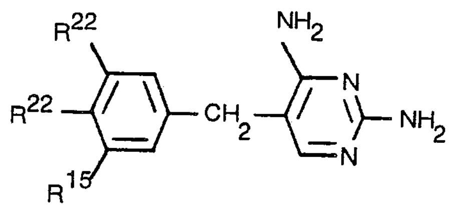

(XVII)

wherein one group $R^{22}$ is a group $—O(CH_2)_nQ$ whilst the other group $R^{22}$ is a group $R^6$ as hereinbefore defined in claim 1 other than hydroxy, $R^{15}$ is as hereinbefore defined in claim 13 and Q is a halogen atom, with a compound of the formula $R^7$ PH wherein $R^7$ is as hereinbefore defined in claim 1 and P is an oxygen or sulphur atom or $CH_2O$ or $CH_2S$ group, and thereafter when $R^{15}$ is a benzyloxy group optionally converting this to a hydroxy group by conventional methods known in the art, optionally followed by the conversion of one group $R^5$, $R^6$ to another group $R^5$, $R^6$ by methods known per se.

**Patentansprüche**

1. Benzylpyrimidin der allgemeinen Formel:

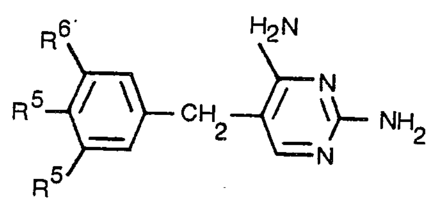

(IV)

oder ein Säureadditionssalz davon, worin eine der Gruppen $R^5$ eine Gruppe $—O(CH_2)_nYR^7$ ist, wobei Y ein Sauerstoff- oder Schwefelatom oder eine Methylen-, $OCH_2$– oder $SCH_2$-Gruppe ist, n eine ganze Zahl von 1 bis 5 und $R^7$ eine gegebenenfalls durch einen oder mehrere Substituenten, ausgewählt aus $C_{1-4}$ Alkoxy, $C_{1-4}$ Alkyl, Trifluormethyl, Nitro, Amino, Methylamino, Dimethylamino oder Acetamidogruppe, substituierte Phenylgruppe bedeutet, die andere Gruppe $R^5$ und $R^6$, die gleich oder verschieden sind, jede Wasserstoff, Halogen, $C_{2-4}$ Alkenyl, $C_{2-4}$ Alkenyloxy, Nitro, Cyano, Hydroxy, Benzyloxy, $C_{1-4}$ Alkyl oder $C_{1-4}$ Alkoxy bedeutet und die Alkyl- oder Alkoxygruppe ggf, durch Halogen, Hydroxy oder $C_{1-2}$ Alkoxy substituiert ist; ggf. eine durch eine oder zwei $C_{1-4}$ Alkyl- oder $C_{1-4}$ Acylgruppen substituierte Aminogruppe oder das Stickstoffatom ist Teil eines fünf- oder sechsgliedrigen heterocyclischen Rings, eine Gruppe $—OSO_2R^8$ oder $S(O)_rR^8$, wobei $R^8$ eine $C_{1-3}$ Alkylgruppe und r 0, 1 oder 2 ist, eine Gruppe $—COR^9$, wobei $R^9$ Methyl, Ethyl, Methoxy, Ethoxy, Amino, Methylamino, Ethylamino, Dimethylamino oder Diethylamino ist.

2. Verbindung nach Anspruch 1, worin eine der Gruppen $R^5$ eine Gruppe $—O(CH_2)_nYR^7$ bedeutet, wobei Y ein Sauerstoff- oder Schwefelatom oder eine Methylen-, $OCH_2$— oder $SCH_2$-Gruppe, n eine ganze Zahl von 1 bis 5 ist und $R^7$ eine ggf. durch einen oder mehrere Substituenten, ausgewählt aus $C_{1-4}$ Alkoxy-, $C_{1-4}$ Alkyl-, Trifluormethyl-, Nitro-, Amino-, Methylamino-, Dimethylamino- oder Acetamidogruppen substituierte Phenylgruppe bedeutet und die andere Gruppe $R^5$ ein Wasserstoff oder Halogenatom oder eine Hydroxy-, Benzyloxy-, $C_{1-4}$ Alkoxy-, $C_{1-4}$ Alkyl- oder $C_{2-4}$ Alkenylgruppe bedeutet und $R^6$ ein

Wasserstoff- oder Halogenatom oder eine Hydroxy, $C_{1-4}$ Alkoxy, $C_{1-4}$ Alkyl oder $C_{2-4}$ Alkenylgruppe bedeutet.

3. Benzylpyrimidin der allgemeinen Formel:

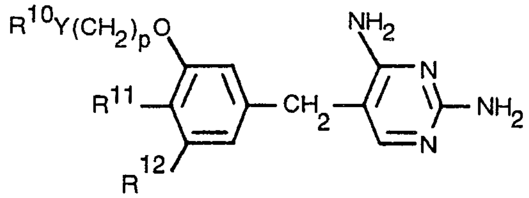

(V)

oder ein Säureadditionssalz davon, worin

p 2 oder 3 ist,

Y ein Sauerstoffatom oder eine Methylengruppe ist,

$R^{10}$ eine Benzylgruppe oder eine ggf. durch eine $C_{1-4}$ Alkoxygruppe substituierte Phenylgruppe bedeutet,

$R^{11}$ ein Iodatom oder eine Methoxy-, Ethoxy- oder Ethylgruppe bedeutet und

$R^{12}$ ein Iodatom oder eine Methoxy-, Ethoxy-, Ethyl-, Propyl-, Propenyl- oder Allylgruppe bedeutet.

4. Benzylpyrimidin der allgemeinen Formel:

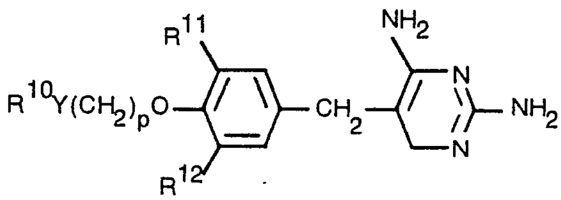

(VI)

oder ein Säureadditionssalz davon, worin

p 2 oder 3 ist,

Y ein Sauerstoffatom oder eine Methylengruppe ist,

$R^{10}$ eine Benzylgruppe oder eine ggf. durch eine $C_{1-4}$ Alkoxygruppe substituierte Phenylgruppe

$R^{11}$ ein Iodatom oder eine Methoxy-, Ethoxy- oder Ethylgruppe bedeutet und

$R^{12}$ ein Iodatom oder eine Methoxy-, Ethoxy-, Ethyl-, Propyl-, Propenyl- oder Allylgruppe bedeutet.

5. Benzylpyrimidin der allgemeinen Formel:

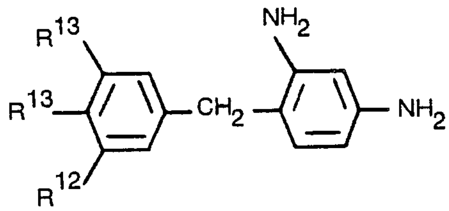

(VII)

oder ein Säureadditionssalz davon, worin eine der Gruppen $R^{13}$ eine Gruppe $—O(CH_2)_{p-1}—YCH_2R^{10}$ bedeutet und die andere Gruppe $R^{13}$ eine Gruppe $R^{11}$ bedeutet, wobei $R^{10}$, $R^{11}$, $R^{12}$, p und Y die für die Formel (V) in Anspruch 3 gegebene Bedeutung aufweisen.

6. Verbindung nach Anspruch 3 bis 5, worin p 2 ist, $R^{10}$ eine Benzyl-, Phenyl- oder (m-Methoxy)phenylgruppe bedeutet und $R^{11}$ und $R^{12}$ Methoxy- oder Ethylgruppen sind.

7. Benzylpyrimidin, ausgewählt aus:

2,4-Diamino-5-(3,4-dimethoxy-5-(4-phenyl-n-butoxy)benzyl)pyrimidin,

2,4-Diamino-5-(3,4-dimethoxy-5-(3-(m-methoxyphenyl)-n-propoxy)benzyl)pyrimidin,

2,4-Diamino-5-(3,4-dimethoxy-5-(3-phenyl-n-propoxy)benzyl)pyrimidin,

2,4-Diamino-5-(3,4-dimethoxy-5-(4-(p-aminophenyl)-n-butoxy)benzyl)pyrimidin,

2,4-Diamino-5-(3,4-dimethoxy-5-(4-(p-nitrophenyl)-n-butoxy)benzyl)pyrimidin,

2,4-Diamino-5-(3,4-dimethoxy-5-(2-benzyloxyethoxy)benzyl)pyrimidin,

2,4-Diamino-5-(3,4-dimethoxy-5-(4-phenoxy-n-butoxy)benzyl)pyrimidin,

2,4-Diamino-5-(3,4-dimethoxy-5-(2-phenylthio)ethoxy)benzyl)pyrimidin,

2,4-Diamino-5-(3,4-dimethoxy-5-(2-(benzylthio)ethoxy)benzyl)pyrimidin,

oder ein Säureadditionssalz davon.

8. Pharmazeutische Zusammensetzung, enthaltend ein Benzylpyrimidin der Formel (IV), wie in Anspruch 1 definiert, in Kombination mit einem pharmazeutisch verträglichen Trägermaterial.

9. Pharmazeutische Zusammensetzung nach Anspruch 8, worin die Zusammensetzung zusätzlich ein Sulphonamid, das ein p-Aminobenzoesäurekompetitor ist, enthält.

10. Pharmazeutische Zusammensetzung nach Anspruch 8 oder 9, die ein Benzylpyrimidin der Formel (IV), wie in Anspruch 1 definiert und ein Sulfadiazin in einem Verhältnis von 1:2 enthält.

11. Pharmazeutische Zusammensetzung nach Anspruch 8 oder 9, die ein Benzylpyrimidin der Formel (IV), wie in Anspruch 1 definiert und ein Sulfamethoxazol in einem Verhältnis von 1:2 bis 1:6 enthält.

12. Benzylpyrimidin der Formel (IV), wie in Anspruch 1 definiert, zur Verwendung in der Human- und Veterinärmedizin.

13. Verfahren zur Herstellung eines Benzylpyrimidins der Formel (IV), wie in Anspruch 1 definiert, wobei das Verfahren besteht aus:

(a) (i) der Reaktion einer Verbindung der Formel (VIII):

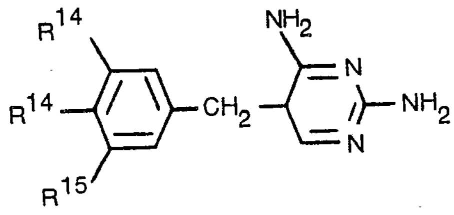

(VIII)

worin eine der Gruppen $R^{14}$ eine Hydroxygruppe ist, während die andere Gruppe $R^{14}$ und $R^{15}$ gleich oder verschieden sind und jede eine Gruppe $R^6$, wie in Anspruch 1 definiert, verschieden von Hydroxy, ist mit einer Verbindung der Formel $R^7Y(CH_2)_nU$, worin n, Y und $R^7$ die in Anspruch 1 gegebene Bedeutung besitzen und U eine austretende Gruppe bedeutet;

(ii) aus der Reaktion von Guanidin in Form des Guanidinsalzes mit einer Verbindung der Formel (IX) oder (X):

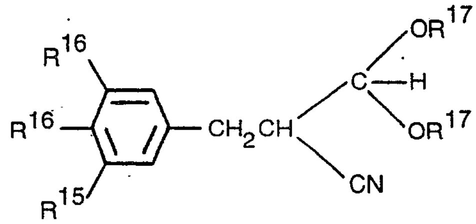

(IX)

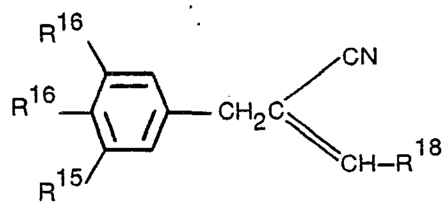

(X)

worin $R^{15}$ die vorhin gegebene Bedeutung aufweist, eine Gruppe $R^{16}$ eine Gruppe $—O(CH_2)_nYR^7$, wie in Anspruch 1 definiert, bedeutet und die andere Gruppe $R^{16}$ eine Gruppe $R^6$, wie in Anspruch 1 definiert ist, $R^{17}$ eine $C_{1-4}$ Alkylgruppe und $R^{18}$ eine $C_{1-4}$ Alkoxygruppe oder eine Amino-, $C_{1-4}$ Alkylamino-, Benzylamino, di-$(C_{1-4})$Alkylamino-, Naphthylamino-, ggf. substituierte Anilino-, Piperidino- oder N-Methylpiperazinogruppe bedeutet; ggf. gefolgt von der Umwandlung einer Gruppe $R^5$, $R^6$ in eine andere Gruppe $R^5$, $R^6$ nach bekannten Verfahren.

14. Verfahren zur Herstellung eines Benzylpyrimidins der Formel (IV), wie in Anspruch 1 definiert, worin Y jedoch keine Alkenylengruppe bedeutet, wobei das Verfahren aus der Reaktion der Verbindung der Formel

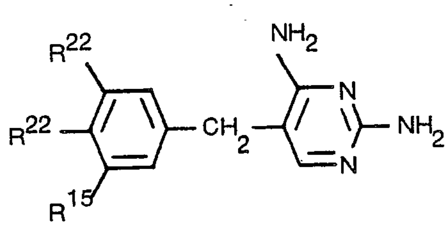

(XVII)

besteht,

worin eine Gruppe $R^{22}$ eine Gruppe $—O(CH_2)_nQ$ bedeutet, während die andere Gruppe $R^{22}$ eine Gruppe $R^6$,

wie in Anspruch 1 definiert, dedeutet, die verschieden von Hydroxy ist, $R^{15}$ die in Anspruch 13 gegebene Bedeutung aufweist und Q ein Halogenatom bedeutet,
mit einer Verbindung der Formel $R^7$ PH worin $R^7$ die in Anspruch 1 gegebene Bedeutung aufweist und P ein Sauerstoff oder Schwefelatom oder eine $CH_2O$ oder $CH_2S$-Gruppe bedeutet, und
anschließend, wenn $R^{15}$ eine Benzyloxy-Gruppe ist, ggfg. in der Überführung dieser Gruppe in eine Hydroxygruppe durch bekannte Verfahren,
ggf. gefolgt von der Umwandlung einer der Gruppen $R^5$, $R^6$ in eine andere Gruppe $R^5$, $R^6$ durch bekannte Verfahren.

**Revendications**

1. Benzylpyrimidine de formule générale:

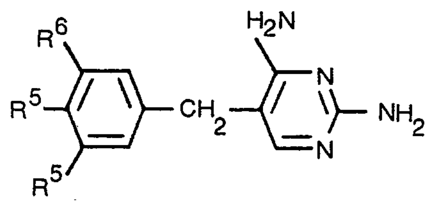

(IV)

ou sel d'addition d'acide de celle-ci,
où l'un des radicaux $R^5$ est un radical $O(CH_2)_nYR^7$ où Y est un atome d'oxygène ou de soufre ou un radical méthylène, $OCH_2$ ou $SCH_2$, n est un nombre entier de 1 à 5 et $R^7$ est un radical phényle éventuellement substitué par un ou plusieurs substituants choisis parmi les radicaux $C_{1-4}$-alcoxy, $C_{1-4}$-alcoyle, trifluorométhyle, nitro, amino, méthylamino, diméthylamino et acétamido; l'autre radical $R^5$ et $R^6$ étant identiques ou différents et étant chacun un atome d'hydrogène ou d'halogène ou radical $C_{2-4}$-alcényle, $C_{2-4}$-alcényloxy, nitro, cyano, hydroxyle, benzyloxy, $C_{1-4}$-alcoyle ou $C_{1-4}$-alcoxy, le radical alcoyle ou alcoxy étant éventuellement substitué par halogène, hydroxyle ou $C_{1-2}$-alcoxy; amino éventuellement substitué par un ou deux radicaux $C_{1-4}$-alcoyle ou $C_{1-4}$-acyle ou l'atome d'azote faisant partie d'un hétérocycle à cinq ou six chaînons, un radical $—OSO_2R^8$ ou $S(O)rR^8$ où $R^8$ est un radical $C_{1-3}$-alcoyle et r représente 0, 1 ou 2, un radical $—COR^9$ où $R^9$ est un radical méthyle, éthyle, méthoxy, éthoxy, amino, méthylamino, éthylamino, diméthylamino ou diéthylamino.

2. Composé suivant la revendication 1, dans lequel l'un des radicaux $R^5$ est un radical $—O(CH_2)_nYR^7$ où Y est un atome d'oxygène ou de soufre ou un radical méthylène, $OCH_2$ ou $SCH_2$, n est un nombre entier de 1 à 5 et $R^7$ est un radical phényle éventuellement substitué par un ou plusieurs substituants choisis parmi les radicaux, $C_{1-4}$-alcoxy, $C_{1-4}$-alcoyle, trifluorométhyle, nitro, amino, méthylamino, diméthylamino et acétamido, l'autre radical $R^5$ est un atome d'hydrogène ou d'halogène ou un radical hydroxyle, benzyloxy, $C_{1-4}$-alcoxy, $C_{1-4}$-alcoyle ou $C_{2-4}$-alcényle, et $R^6$ est un atome d'hydrogène ou d'halogène ou un radical hydroxyle, $C_{1-4}$-alcoxy, $C_{1-4}$-alcoyle ou $C_{2-4}$-alcényle.

3. Benzylpyrimidine de la formule générale:

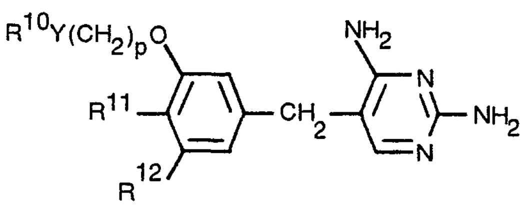

(V)

ou sel d'addition d'acide de celle-ci,
où

p représente 1 ou 2,
Y est un atome d'oxygène ou un radical méthylène,
$R^{10}$ est un radical benzyle ou un radical phényle éventuellement substitué par un radical $C_{1-4}$-alcoxy,
$R^{11}$ est un atome d'iode ou un radical méthoxy, éthoxy ou éthyle, et
$R^{12}$ est un atome d'iode ou un radical méthoxy, éthoxy, éthyle, propyle, propényle ou allyle.

4. Benzylpyrimidine de formule générale:

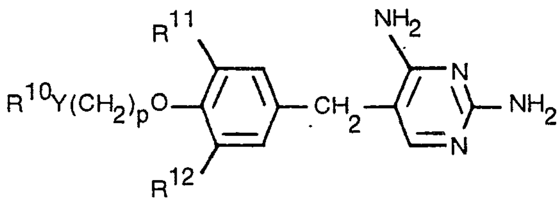

(VI)

ou sel d'addition d'acide de celle-ci,

p représente 2 ou 3,

Y est un atome d'oxygène ou un radical méthylène,

$R^{10}$ est un radical benzyle ou un radical phényle éventuellement substitué par un radical $C_{1-4}$-alcoxy,

$R^{11}$ est un atome d'iode ou un radical méthoxy, éthoxy ou éthyle, et

$R^{12}$ est un atome d'iode ou un radical méthoxy, éthoxy, éthyle, propyle, propényle ou allyle.

5. Benzylpyrimidine de formule générale:

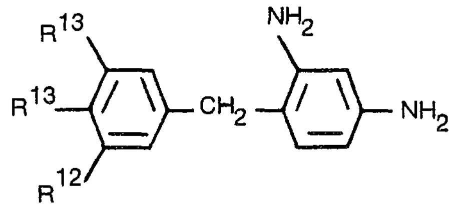

(VII)

ou sel d'addition d'acide de celle-ci, où l'un d'entre les radicaux $R^{13}$ est un radical $O(CH_2)_{p-1}YCH_2R^{10}$ et l'autre radical $R^{13}$ est un radical $R^{11}$; où $R^{10}$, $R^{11}$, $R^{12}$, p et Y sont tels que définis à propos de la formule (V) dans la revendication 3.

6. Composé suivant l'une quelconque des revendications 3 à 5, dans lequel

p représente 2,

$R^{10}$ est un radical benzyle, phényle ou (m-méthoxy)phényle et

$R^{11}$ et $R^{12}$ sont des radicaux méthoxy ou éthyle.

3. Benzylpyrimidine choisie parmi:

la 2,4-diamino-5-(3,4-diméthoxy-5-(4-phényl-n-butoxy)benzyl)pyrimidine,

la 2,4-diamino-5-(3,4-diméthoxy-5-(3-(m-méthoxyphényl)-n-propoxy)benzyl)pyrimidine,

la 2,4-diamino-5-(3,4-diméthoxy-5-(3-phényl-n-propoxy)benzyl)pyrimidine,

la 2,4-diamino-5-(3,4-diméthoxy-5-(4-(p-aminophényl)-n-butoxy)benzyl)pyrimidine,

la 2,4-diamino-5-(3,4-diméthoxy-5-(4-(p-nitrophényl)-n-butoxy)benzyl)pyrimidine,

la 2,4-diamino-5-(3,4-diméthoxy-5-(2-benzyloxyéthoxy)benzyl)pyrimidine,

la 2,4-diamino-5-(3,4-diméthoxy-5-(4-phénoxy-n-butoxy)benzyl)pyrimidine,

la 2,4-diamino-5-(3,4-diméthoxy-5-(2-phénylthio)éthoxy)benzyl)pyrimidine,

la 2,4-diamino-5-(3,4-diméthoxy-5-(2-benzylthio)éthoxy)benzyl)pyrimidine,

ou sel d'addition d'acide de celle-ci.

8. Composition pharmaceutique comprenant une benzylpyrimidine de la formule (IV) telle que définie dans la revendication 1, en combinaison avec un excipient pharmaceutiquement acceptable.

9. Composition pharmaceutique suivant la revendication 8, dans laquelle la composition comprend, en outre, un sulfonamide qui est un agent entrant en compétition avec l'acide p-aminobenzoïque.

10. Composition pharmaceutique suivant la revendication 8 ou 9, qui comprend une benzylpyrimidine de la formule (IV) telle que définie dans la revendication 1, et de la sulfadiazine dans un rapport de 1:2.

11. Composition pharmaceutique suivant la revendication 8 ou 9, qui comprend une benzylpyrimidine de la formule (IV) telle que définié dans la revendication 1, et du sulfaméthoxazole dans un rapport de 1:2 à 1:6.

12. Benzylpyrimidine de la formule (IV) telle que définie dans la revendication 1, à utiliser en médecines humaine et vétérinaire.

13. Procédé de préparation d'une benzylpyrimidine de la formule (IV) telle que définie dans la revendication 1, lequel procédé comprend:

(a) (i) la réaction d'un composé de formule (VIII):

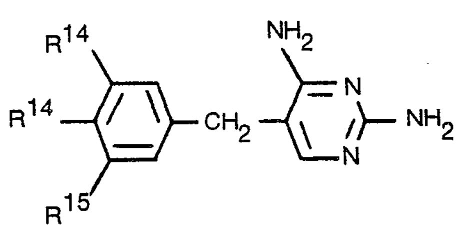

(VIII)

où un radical $R^{14}$ est un radical hydroxyle, tandis que l'autre radical $R^{14}$ et $R^{15}$ sont identiques ou différents et sont chacun un radical $R^6$ tel que défini ci-dessus dans la revendication 1 autre qu'un radical hydroxyle, avec un composé de formule $R^7Y(CH_2)_nU$,
où n, Y et $R^7$ sont tels que définis ci-dessus dans la revendication 1 et U est un radical partant,

(ii) la réaction de la guanidine à l'état de sel de guanidine avec un composé de formule (IX) ou (X):

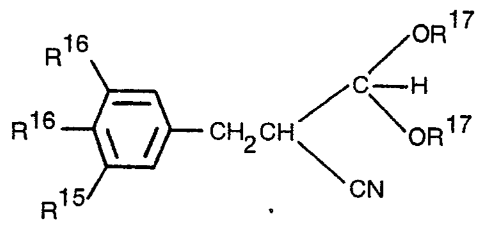

(IX)

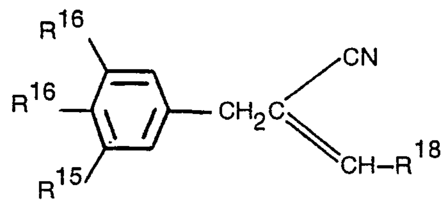

(X)

où $R^{15}$ est tel que défini ci-dessus, un radical $R^{16}$ est un radical $O(CH_2)_nYR^7$ tel que défini ci-dessus dans la revendication 1 et l'autre radical $R^{16}$ est un radical $R^6$ tel que défini ci-dessus dans la revendication 1, $R^{17}$ est un radical $C_{1-4}$-alcoyle et $R^{18}$ est un radical $C_{1-4}$-alcoxy ou un radical amino, $C_{1-4}$-alcoylamino, benzylamino, di-$(C_{1-4})$alcoylamino, naphtylamino, anilino éventuellement substitué, pipéridino ou N-méthylpipérazino,
éventuellement suivie de la conversion d'un radical $R^5$, $R^6$ en un autre radical $R^5$, $R^6$ suivant des procédés connus.

14. Procédé de préparation d'une benzylpyrimidine de la formule (IV) telle que définie dans la revendication 1, mais où Y n'est pas un radical alcoylène, lequel procédé comprend la réaction d'un composé de formule:

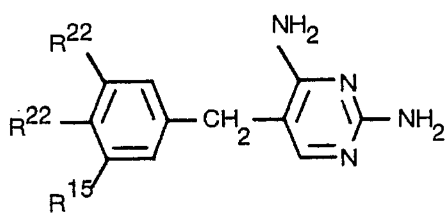

(XVII)

où un radical $R^{22}$ est un radical $—O(CH_2)_nQ$, tandis que l'autre radical $R^{22}$ est un radical $R^6$ tel que défini ci-dessus dans la revendication 1 autre qu'un radical hydroxyle, $R^{15}$ est tel que défini ci-dessus dans la revendication 13 et Q est un atome d'halogène, avec un composé de la formule $R^7PH$ où $R^7$ est tel que défini ci-dessus dans la revendication 1 et P est un atome d'oxygène ou de soufre ou un radical $CH_2O$ ou $CH_2S$ et ensuite, lorsque $R^{15}$ est un radical benzyloxy, la conversion éventuelle de ce dernier en un radical hydroxyle suivant des procédés connus,
éventuellement suivie de la conversion d'un radical $R^5$, $R^6$ en un autre radical $R^5$, $R^6$ suivant des procédés connus.